# EUROPEAN PATENT APPLICATION

(11) **EP 2 926 713 A1**
(43) Date of publication of application: **07.10.2015**
(21) Application number: 13859294.4
(22) Date of filing: 22.11.2013
(51) Int. Cl.: A61B 1/00, A61B 1/04, G01N 21/64, G02B 23/24, G02B 23/26

(54) **OBSERVATION DEVICE**

(30) Priority: 30.11.2012 JP 2012262498
(71) Applicant: Olympus Corporation, Shibuya-ku Tokyo 151-0072 (JP)
(72) Inventor: KUBO, Kei, Tokyo 151-0072 (JP); ISHIHARA, Yasushige, Tokyo 151-0072 (JP); SHIDA, Hiromi, Tokyo 151-0072 (JP)
(74) Representative: Schicker, Silvia
(86) International application number: PCT/JP2013/081496
(87) International publication number: WO 2014/084134

(57) **Abstract**

Provided is an observation apparatus (100) comprising a light source (3) that irradiates a subject (X) with illumination light and special light that acts on a specific region of the subject (X); a return-light-image generating portion (61) that generates a return-light image (G1) by capturing return light coming from the subject (X) due to irradiation with the illumination light; a special-light-image generating portion (62) that generates a special-light image (G2) by capturing signal light coming from the subject (X) due to irradiation with the special light; an extraction portion (63) that extracts the specific region from the special-light image (G2); and an enhancement processing portion (64) that performs enhancement processing, which is based on return-light image information, on the return-light image (G1), in a region corresponding to the extracted specific region.

## Description

### {Technical Field}

The present invention relates to an observation apparatus.

### {Background Art}

In the related art, there are known observation apparatuses that selectively capture images of a region-of-interest, such as a lesion, in a subject by using light of a specific wavelength, that identify the position of the region-of-interest by using an obtained special-light image, and that label the identified position in a white-light image with a marker (for example, see Patent Literature 1). With the marker which is displayed in the white-light image, the user can easily recognize the region-of-interest that exists in the observation field of view.

### {Citation List}

### {Patent Literature}

{PTL 1} Japanese Unexamined Patent Application, Publication No. 2011-104011

### {Summary of Invention}

### {Technical Problem}

For the user to correctly diagnose the region-of-interest, it is necessary to perform detailed examination of the morphology of the region-of-interest by using the white-light image. However, as described in Patent Literature 1, if the marker is displayed at a position that interferes with the region-of-interest, there is a problem in that detailed examination of the region-of-interest is hindered by this marker.

The present invention has been conceived in light of the above-described situation, and an object thereof is to provide an observation apparatus with which it is possible to easily recognize a region-of-interest, such as a lesion, that exists in an observation field of view, and with which it is possible to perform a detailed examination of the morphology of the region-of-interest based on a white-light image.

### {Solution to Problem}

In order to realize the above object, the present invention provides the following solutions.

The present invention provides an observation apparatus including a light source that irradiates a subject with illumination light and special light in a wavelength band different from that of the illumination light, which acts on a specific region of the subject; a return-light-image generating portion that generates a return-light image by capturing return light emitted from the subject due to irradiation with the illumination light from the light source; a special-light-image generating portion that generates a special-light image by capturing signal light emitted from the subject due to irradiation with the special light from the light source; an extraction portion that extracts the specific region from the special-light image generated by the special-light-image generating portion; and an enhancement processing portion that performs enhancement processing, which is based on return-light image information, on the return-light image generated by the return-light-image generating portion, in a region corresponding to the specific region extracted by the extraction portion.

According to the present invention, by irradiating the subject with illumination light from a light source, a return-light image formed by capturing the morphology of the subject is obtained by the return-light-image generating portion. On the other hand, by irradiating the subject with special light from the light source, a special-light image formed by capturing a specific region, such as a lesion, that exists in the subject is obtained by the special-light-image generating portion. Then, the specific region in the special-light image is extracted by the extraction portion, and the specific region in the return-light image is subjected to enhancement processing by the enhancement processing portion. In this way, the user can observe the detailed morphology of a region-of-interest while still being able to easily recognize the region-of-interest that exists in the observation field of view by using the white-light image in which the specific region-of-interest has been subjected to enhancement processing.

In the above-described invention, the enhancement processing portion may enhance the contrast of at least one of structure and color.

By doing so, a peripheral region, such as a normal area of the subject is not changed from the structure and/or color of the unprocessed white-light image, and the structure and/or color contrast of only the region-of-interest, such as a lesion, in the subject are enhanced, so that an image in which the lesion is provided with noticeable characteristics is obtained. In addition, since the structure and/or color contrast of the region-of-interest in this image are enhanced compared with those in the unprocessed white-light image, it is possible to observe the morphology of the region-of-interest in detail, and it is possible to effectively enhance the region-of-interest.

In the above-described invention, as the special light, the light source may radiate excitation light that excites a fluorescent substance contained in the specific region, and the special-light-image generating portion may generate, as the special-light image, a fluorescence image by capturing fluorescence from the fluorescent substance.

By doing so, it is possible to identify the region-of-interest on the basis of the distribution of the fluorescent substance.

In the above-described invention, the extraction portion may extract, as the specific region, a region having a gradation value or hue equal to or higher than a prescribed threshold.

By doing so, it is possible to extract the specific region by means of simple processing.

The above-described invention may further include an enhancement-level setting portion that sets, for the specific region, a degree of enhancement to be performed by the enhancement processing portion, on the basis of the gradation value or hue of the specific region extracted by the extraction portion.

By doing so, it is possible to appropriately enhance the specific region according to the degree of difference in color or structure of the specific region relative to the surrounding region.

In the above-described invention, the light source may radiate narrow-band light as the special light, and the special-light-image generating portion may generate, as the special-light image, a narrow-band-light (NBI) image by capturing return light from the subject by irradiation with the narrow-band light.

By doing so, it is possible to identify the region-of-interest on the basis of the difference in concentration of blood vessels in the subject.

In the above-described invention, the light source may radiate, as the special light, excitation light that excites autofluorescence in a substance contained in the subject, and the special-light-image generating portion may generate, as the special-light image, an autofluorescence image by capturing autofluorescence from the substance.

By doing so, it is possible to identify the region-of-interest on the basis of the intensity distribution of autofluorescence emitted from the subject.

The above-described invention may further include a combining portion that combines a marker showing the specific region extracted by the extraction portion with the return-light image generated by the return-light-image generating portion; a display portion that displays an image; a determination portion that determines an observation distance to the subject; and a display switching portion that selectively displays, on the display portion, a return-light image in which the specific region is enhanced by the enhancement processing portion and a return-light image with which the marker is combined by the combining portion, on the basis of the observation distance determined by the determination portion.

By doing so, when observing the subject from a distant position, a return-light image in which the marker is combined with the specific region is displayed on the display portion. Accordingly, even if the dimensions of the specific region in the return-light image are small, it is possible to reliably recognize the existence of the specific region. On the other hand, when observing the subject from a close position, a return-light image in which the specific region has been subjected to enhancement processing is displayed on the display portion. Accordingly, it is possible to observe the morphology of the specific region in detail.

In the above described invention, the determination portion may determine the observation distance by using a gradation value of the return-light image generated by the return-light-image generating portion, or may determine the observation distance by using an area, in the special-light image, of the specific region extracted by the extraction portion.

By doing so, it is possible to appropriately determine the observation distance with computational processing alone.

### {Advantageous Effects of Invention}

The present invention affords an advantage in that it is possible to easily recognize a region-of-interest, such as a lesion, that is present in an observation field of view, while also allowing detailed observation of the morphology of the region-of-interest based on a white-light image.

### {Brief Description of Drawings}

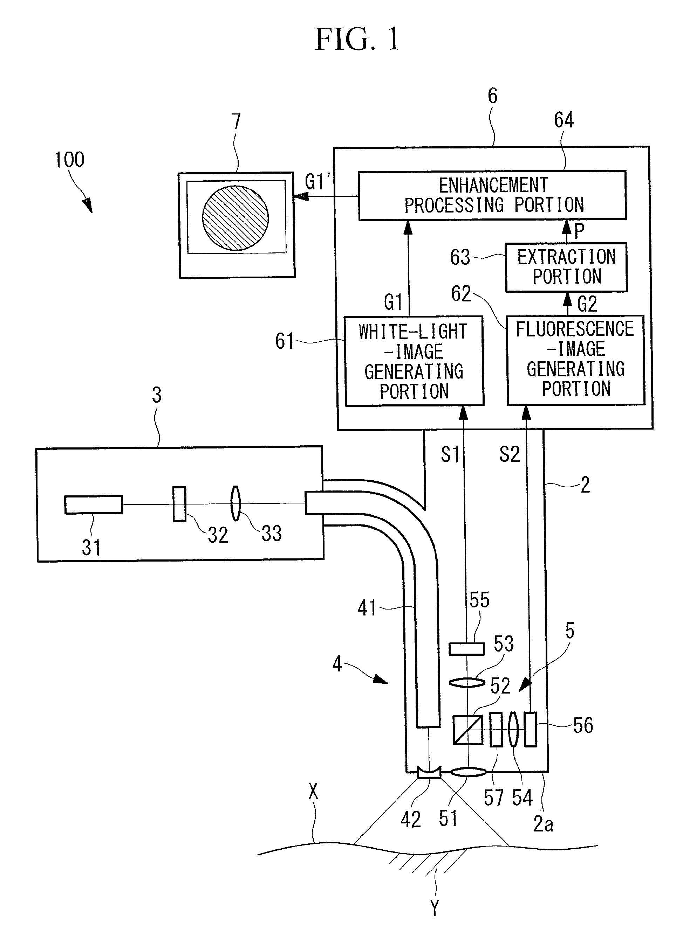

{Fig. 1}
   Fig. 1 is a diagram showing the overall configuration of an observation apparatus according to a first embodiment of the present invention.
{Fig. 2}
   Fig. 2 is a flowchart showing image processing performed by the observation apparatus in Fig. 1.
{Fig. 3}
   Fig. 3 is a diagram showing the configuration of an image processing unit provided in an observation apparatus according to a second modification of the first embodiment.
{Fig. 4}
   Fig. 4 is a diagram showing the configuration of an image processing unit provided in an observation apparatus according to a third modification of the first embodiment.
{Fig. 5}
   Fig. 5 is a graph showing a function relating a mean gradation value and a degree of enhancement processing, which is used in an enhancement-level setting portion in Fig. 4.
{Fig. 6}
   Fig. 6 is a flowchart for explaining image processing performed by the image processing unit in Fig. 4.
{Fig. 7}
   Fig. 7 is a diagram showing the configuration of an image processing unit provided in an observation apparatus according to a fourth modification of the first embodiment.
{Fig. 8}
   Fig. 8 is a flowchart for explaining image processing performed by the image processing unit in Fig. 7.
{Fig. 9}
   Fig. 9 is a diagram showing the overall configuration of an observation apparatus according to a second embodiment of the present invention.
{Fig. 10}
   Fig. 10 is a diagram showing the configuration of an image processing unit provided in an observation apparatus according to a modification of the second embodiment.
{Fig. 11}
   Fig. 11 is a diagram showing the overall configuration of an observation apparatus according to a third embodiment of the present invention.
{Fig. 12}
   Fig. 12 is a diagram showing the overall configuration of an observation apparatus according to a fourth embodiment of the present invention.

### {Description of Embodiments}

### First Embodiment

An observation apparatus 100 according to a first embodiment of the present invention will be described below with reference to Figs. 1 to 8.

The observation apparatus 100 according to this embodiment is an endoscope apparatus and, as shown in Fig. 1, includes an elongated insertion portion 2 for insertion into a body; a light source 3; an illumination unit 4 that radiates excitation light (special light) and white light (illumination light) from the light source 3 towards an observation target (subject) X from a distal end 2a of the insertion portion 2; an image-acquisition unit 5 that obtains image information S1 and S2 of biological tissue, that is, the observation target X; an image processing unit 6 that is disposed at the base end of the insertion portion 2 and that processes the image information S1 and S2 obtained by the image-acquisition unit 5; and a monitor (display portion) 7 that displays an image G1' processed by the image processing unit 6.

The light source 3 includes a xenon lamp 31, a filter 32 that extracts excitation light and white light from the light emitted from the xenon lamp 31, and a coupling lens 33 that focuses the excitation light and the white light extracted by the filter 32. The filter 32 selectively transmits light in a wavelength band of 400 nm to 740 nm, corresponding to the excitation light and the white light. In other words, in this embodiment, near-infrared light (wavelength band 700 nm to 740 nm) is used as the excitation light.

The illumination unit 4 includes a light guide fiber 41 that is disposed along substantially the entire length of the insertion portion 2 in the longitudinal direction thereof and an illumination optical system 42 that is provided at the distal end 2a of the insertion portion 2. The light guide fiber 41 guides the excitation light and the white light focused by the coupling lens 33. The illumination optical system 42 spreads out the excitation light and the white light guided thereto by the light guide fiber 41 and irradiates the observation target X, which faces the distal end 2a of the insertion portion 2.

The image-acquisition unit 5 includes an objective lens 51 that collects light coming from the observation target X; a dichroic mirror 52 that reflects the excitation light and fluorescence (signal light) in the light collected by the objective lens 51 and transmits white light having a wavelength shorter than that of the excitation light (wavelength band 400 nm to 700 nm, return light); two focusing lenses 53 and 54 that respectively focus the fluorescence reflected by the dichroic mirror 52 and the white light transmitted through the dichroic mirror 52; an image-acquisition device 55, such as a color CCD, that captures the white light focused by the focusing lens 53; and an image-acquisition device 56, such as a high-sensitivity monochrome CCD, that captures the fluorescence focused by the focusing lens 54. Reference sign 57 in the figure is an excitation-light cutting filter that selectively transmits the fluorescence (wavelength band 760 nm to 850 nm) in the light reflected by the dichroic mirror 52 and blocks the excitation light.

The image processing unit 6 includes a white-light-image generating portion (return-light-image generating portion) 61 that generates a white-light image (return-light image) from the white-light image information S1 obtained by the image-acquisition device 55; a fluorescence-image generating portion (special-light-image generating portion) 62 that generates a fluorescence image (special-light image) G2 from the fluorescence image information S2 obtained by the image-acquisition device 56; an extraction portion 63 that extracts a region-of-interest (specific region), such as a lesion Y, from the fluorescence image G2 generated by the fluorescence-image generating portion 62; and an enhancement processing portion 64 that executes enhancement processing on a region in the white-light image G1 that corresponds to the region-of-interest extracted by the extraction portion 63.

The extraction portion 63 compares the gradation value of each pixel in the fluorescence image G2 input thereto from the fluorescence-image generating portion 62 with a prescribed threshold, extracts pixels having a gradation value equal to or higher than the prescribed threshold as a region-of-interest, and outputs positions P of the extracted pixels to the enhancement processing portion 64.

The enhancement processing portion 64 selects, from the white-light image G1, pixels at positions corresponding to the positions P of the pixels input thereto from the extraction portion 63, enhances the color of the region-of-interest formed of the selected pixels, and outputs a white-light image G1', in which the region-of-interest has been subjected to enhancement processing, to the monitor 7.

More specifically, the enhancement processing portion 64 subjects the white-light image G1 to hemoglobin index (IHb) color enhancement processing. IHb color enhancement is processing in which the color at positions on the mucous membrane covering the surface of biological tissue, that is, the observation target X, where the hemoglobin index is higher than average, is made more red, and the color at positions where the hemoglobin index is lower than the average is made more white. The absorption coefficients of hemoglobin in the green (G) and red (R) wavelength regions are different from each other. By using this fact, the hemoglobin index at each position in the white-light image G1 is measured by calculating the ratio of the brightness levels of a G signal and an R signal from the white-light image information S1.

The lesion Y has a red tinge compared with normal parts around it. This is because the cells are more active and the blood flow is higher in the lesion Y. The color of this lesion Y can be enhanced via IHb color enhancement, which allows the user to perform more detailed examination of the lesion Y.

Next, the operation of the thus-configured observation apparatus 100 will be described.

To observe biological tissue inside a body, that is, the observation target X, by using the observation apparatus 100 according to this embodiment, a fluorescent substance that accumulates in the lesion Y is administered in advance to the observation target X. Then, the insertion portion 2 is inserted into the body so that the distal end 2a of the insertion portion 2 is disposed facing the observation target X. Next, by operating the light source 3, the excitation light and white light are radiated onto the observation target X from the distal end 2a of the insertion portion 2.

Fluorescence is generated in the observation target X as a result of excitation of the fluorescent substance contained in the lesion Y by the excitation light, and the white light is reflected at the surface of the observation target X. Parts of the fluorescence emitted from the observation target X and the white light reflected therefrom return to the distal end 2a of the insertion portion 2 and are collected by the objective lens 51.

Of the light collected by the objective lens 51, the white light is transmitted through the dichroic mirror 52 and is focused by the focusing lens 53, and the white-light image information S1 is obtained by the image-acquisition device 55. On the other hand, the fluorescence collected by the objective lens 51 is reflected by the dichroic mirror 52 and, after the excitation light is removed therefrom by the excitation-light cutting filter 57, is focused by the focusing lens 54, and the fluorescence image information S2 is obtained by the image-acquisition device 56. The image information S1 and S2 obtained by the respective image-acquisition devices 55 and 56 are sent to the image processing unit 6.

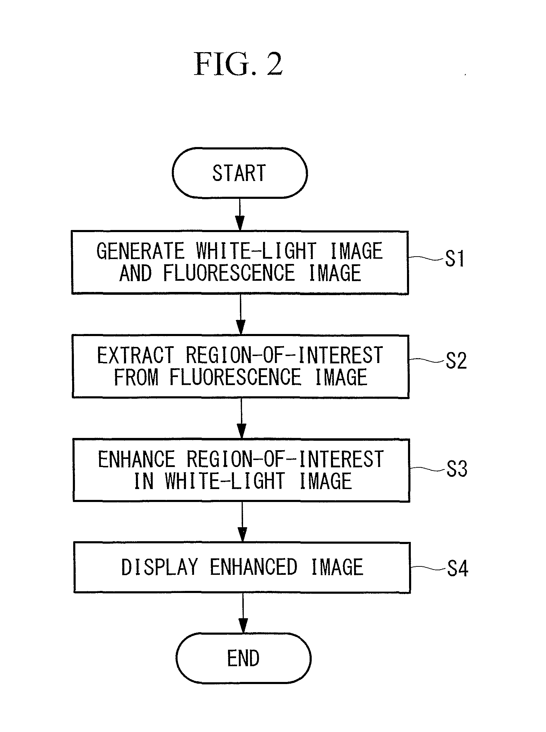

Fig. 2 shows a flowchart for explaining image processing performed by the image processing unit 6.

In the image processing unit 6, the white-light image information S1 is input to the white-light-image generating portion 61, where the white-light image G1 is generated, and the fluorescence image information S2 is input to the fluorescence-image generating portion 62, where the fluorescence image G2 is generated (step S1).

The fluorescence image G2 is sent to the extraction portion 63, where the region-of-interest having gradation values equal to or higher than the prescribed threshold is extracted (step S2). The position P of the extracted region-of-interest is sent from the extraction portion 63 to the enhancement processing portion 64, and the region-of-interest in the white-light image G1 is subjected to color enhancement processing in the enhancement processing portion 64 (step S3). Then, the white-light image G1' in which the region-of-interest has been subjected to enhancement processing is displayed on the monitor 7 (step S4). If a region-of-interest is not extracted in step S2, the unprocessed white-light image G1 is displayed on the monitor 7 in step S4.

The extraction portion 63 in this embodiment may calculate the area of the region-of-interest from the number of pixels constituting the region-of-interest, and for a region-of-interest having an area equal to or larger than a threshold that is set in advance, the positions P of the extracted pixels may be output to the enhancement processing portion 64. By doing so, regions-of-interest having extremely small areas can be removed as noise.

In this way, with this embodiment, when a region-of-interest such as the lesion Y exists in the viewing field of the white-light image G1, that region-of-interest is displayed in an enhanced manner. Therefore, an advantage is afforded in that the user can easily recognize the region-of-interest in the white-light image G1' displayed on the monitor 7, and in addition, he or she can confirm, in detail, the morphology of the region-of-interest by using the white-light image G1'.

### First Modification

Next, a first modification of the observation apparatus 100 according to the first embodiment will be described.

The observation apparatus according to this modification is one in which the details of the processing in the enhancement processing portion 64 of the observation apparatus 100 are modified.

In this modification, the enhancement processing portion 64 enhances the structure of the region-of-interest by extracting the outline of tissue in the region-of-interest from the white-light image G1 and enhancing the outline of the tissue in the region-of-interest. To extract the outline, for example, edge extraction processing such as a differential filter is used. Thus, even when using structure enhancement processing instead of the color enhancement processing described above, it is possible to easily recognize the region-of-interest in the white-light image G1', and the morphology of the region-of-interest can be examined in detail.

In this modification, the enhancement processing portion 64 may perform both structure enhancement processing and color enhancement processing. If the enhancement processing portion 64 is capable of executing both structure enhancement processing and color enhancement processing, an input unit (not illustrated in the drawing) for specifying, in the enhancement processing portion 64, the enhancement processing to be applied to the white-light image G1, via a user selection, may be provided.

### Second Modification

Next, a second modification of the observation apparatus 100 according to the first embodiment will be described.

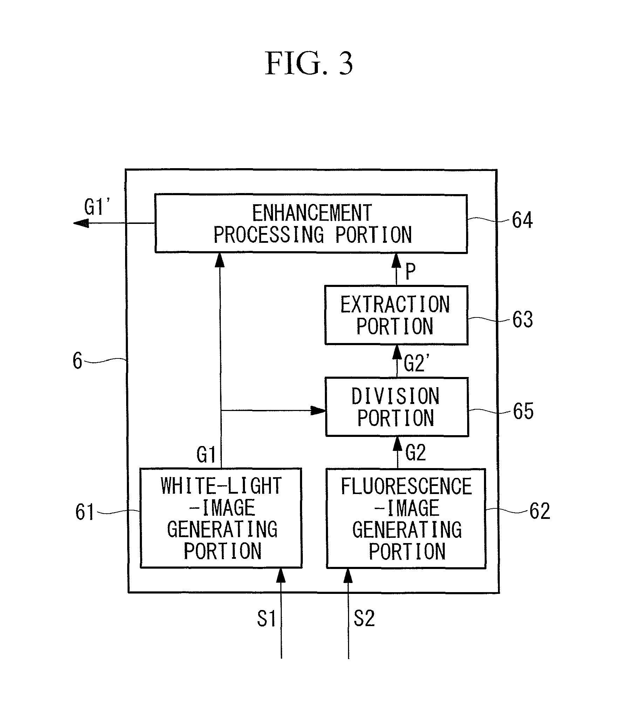

The observation apparatus according to this modification is one in which the image processing unit 6 in the observation apparatus 100 is modified; as shown in Fig. 3, a division portion 65 is further provided in the image processing unit 6.

The division portion 65 receives the white-light image G1 from the white-light-image generating portion 61 and receives the fluorescence image G2 from the fluorescence-image generating portion 62. Then, the division portion 65 generates a division image G2' formed by dividing the fluorescence image G2 by the white-light image G1 and outputs the generated division image G2' to the extraction portion 63. Using the division image G2' instead of the fluorescence image G2, the extraction portion 63 extracts the region-of-interest from the division image G2'.

The gradation values of the fluorescence image G2 depend on the observation distance between the distal end 2a of the insertion portion 2 and the observation target X. In other words, even if it is assumed that the actual intensity of the fluorescence emitted from the observation target X is the same, the gradation values of the fluorescence image G2 become smaller as the observation distance increases. This relationship between the observation distance and the gradation values also holds for the white-light image G1. Thus, dividing the gradation value of each pixel in the fluorescence image G2 by the gradation value of each pixel in the white-light image G1 yields the division image G2', in which the observation-distance-dependent variations in the gradation value in the fluorescence image G2 are removed. In this way, by using the division image G2' which reflects the actual fluorescence intensity more accurately than the fluorescence image G2, an advantage is afforded in that it is possible to extract the region-of-interest more accurately.

### Third Modification

Next, a third modification of the observation apparatus 100 according to the first embodiment will be described.

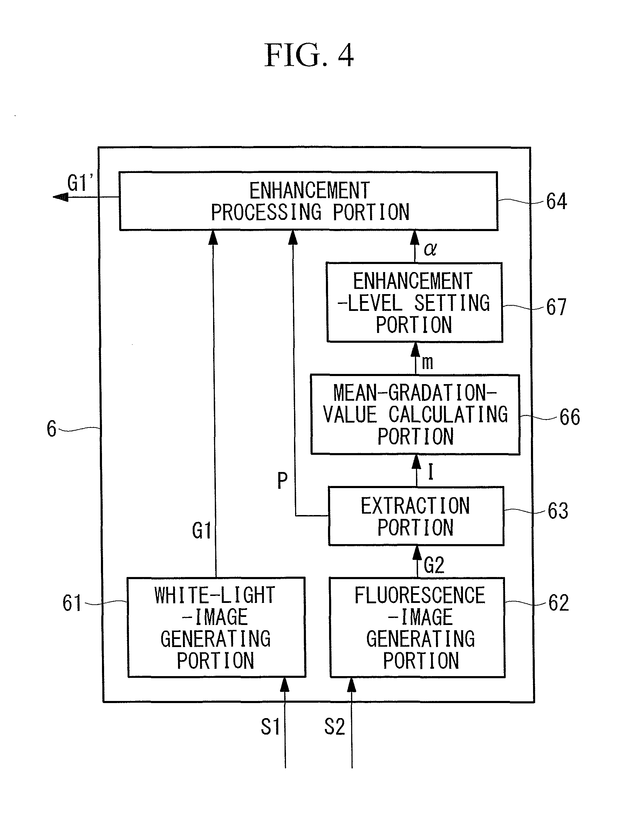

The observation apparatus according to this modification is one in which the image processing unit 6 in the observation apparatus 100 is modified; as shown in Fig. 4, a mean-gradation-value calculating portion 66 and an enhancement-level setting portion 67 are further provided in the image processing unit 6.

In this modification, the extraction portion 63 outputs the positions P of the pixels constituting the region-of-interest to the enhancement processing portion 64 and outputs gradation values I of those pixels to the mean-gradation-value calculating portion 66.

The mean-gradation-value calculating portion 66 calculates a mean m of the gradation values I of the pixels constituting the region-of-interest, extracted by the extraction portion 63, and outputs the calculated mean m of the gradation values I to the enhancement-level setting portion 67.

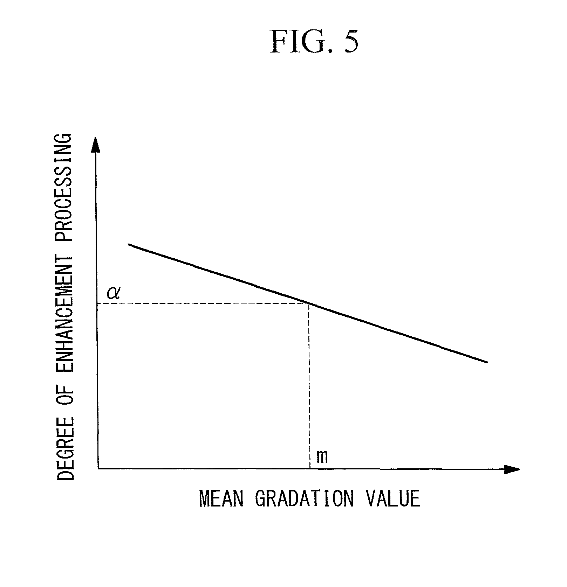

The enhancement-level setting portion 67 sets a degree of enhancement processing, α, in the enhancement processing portion 64 on the basis of the mean m of the gradation values I input from the mean-gradation-value calculating portion 66. More specifically, the enhancement-level setting portion 67 holds a function with which the mean m of the gradation values I and the degree of enhancement processing, α, are associated. As shown in Fig. 5, for example, this function is set so that the degree of enhancement processing, α decreases as the mean m of the gradation values I increases. The enhancement-level setting portion 67 derives the degree of enhancement processing, α, corresponding to the mean m of the gradation values I from the function and outputs the derived degree α to the enhancement processing portion 64.

The enhancement processing portion 64 executes enhancement processing on the region in the white-light image G1 that corresponds to the position P of the region-of-interest input from the extraction portion 63 by using the degree α input from the enhancement-level setting portion 67. That is, even if the hemoglobin indexes are at similar levels relative to the mean, if the degree α is high, the enhancement processing portion 64 subjects the white-light image G1 to IHb color enhancement processing so that the relevant positions are made more red.

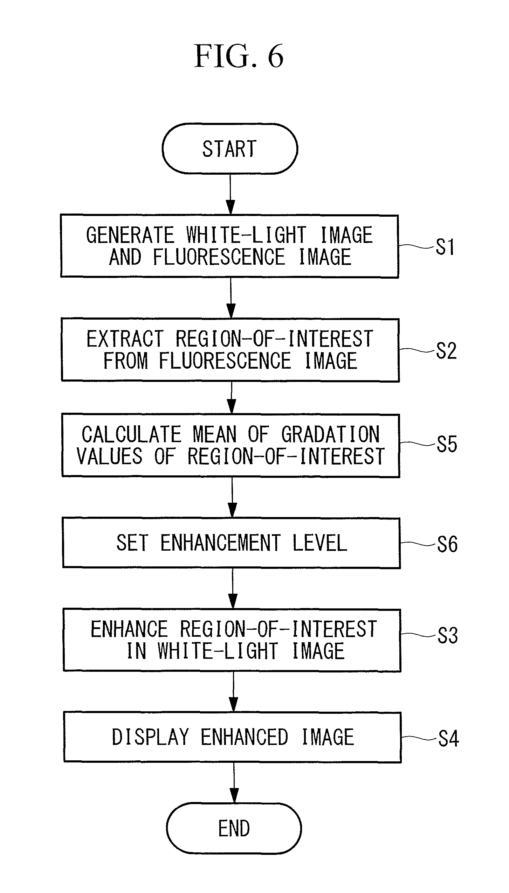

With the thus-configured observation apparatus according to this modification, as shown in Fig. 6, when the region-of-interest is extracted in step S2, the mean m of the gradation values I of that region-of-interest is calculated in the mean-gradation-value calculating portion 66 (step S5). Then, the degree of enhancement processing, α, is determined in the enhancement-level setting portion 67 based on the calculated mean m of the gradation values I (step S6), and the region-of-interest in the white-light image G1 is subjected to enhancement processing in the enhancement processing portion 64 with the determined degree α (step S3).

In this way, by determining the degree of enhancement processing, α, by considering the fluorescence intensities of the entire region-of-interest, when the fluorescence of the region-of-interest becomes weak, the region-of-interest is more strongly enhanced. Accordingly, even for a region-of-interest in which the difference in morphology of the tissue is small relative to the surrounding region, as in an early-stage lesion Y, for example, it can be displayed in a manner sufficiently enhanced with respect to the surrounding region, which affords the advantage that it can be reliably recognized by the user.

The function that associates the mean m of the gradation values I and the degree of enhancement processing, α, may be set such that the degree of enhancement processing, α, increases as the mean m of the gradation values I increases. With this function, when the fluorescence of the region-of-interest is high, the region-of-interest is more strongly enhanced. Accordingly, an advantage is afforded in that the user can reliably recognize a region-of-interest in which the fluorescence intensity is high.

### Fourth Modification

Next, a fourth modification of the observation apparatus 100 according to the first embodiment will be described.

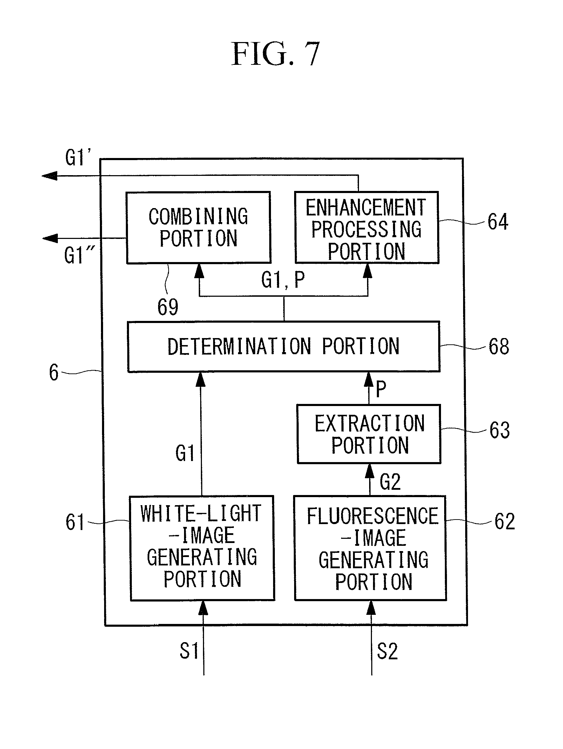

The observation apparatus according to this modification is one in which the image processing unit 6 in the observation apparatus 100 is modified; as shown in Fig. 7, a determination portion (display switching portion) 68 and a combining portion 69 are further provided in the image processing unit 6.

The determination portion 68 determines the observation distance between the observation target X and the distal end 2a of the insertion portion 2 at which the objective lens 51 is disposed, by using the area of the region-of-interest in the fluorescence image G2. More specifically, the determination portion 68 receives the positions P of the pixels constituting the region-of-interest from the extraction portion 63 and calculates the area of the region-of-interest in the fluorescence image G2. The area of the region-of-interest in the fluorescence image G2 increases as the observation distance decreases. Therefore, the determination portion 68 can determine the observation distance from the area of the region-of-interest.

When the calculated area of the region-of-interest is smaller than a prescribed threshold, the determination portion 68 outputs that white-light image G1 input thereto from the white-light-image generating portion 61 to the combining portion 69. On the other hand, when the area of the region-of-interest is equal to or larger than the prescribed threshold, the determination portion 68 outputs the white-light image G1 input thereto from the white-light-image generating portion 61 to the enhancement processing portion 64.

Once the white-light image G1 and the position P of the region-of-interest are input to the combining portion 69 from the determination portion 68, the combining portion 69 creates a marker at the position of the region-of-interest, overlays this marker on the white-light image G1, and outputs a white-light image G1" having the marker overlaid thereon to the monitor 7. The marker is not particularly limited; a marker in which the region-of-interest is filled-in may be used, or a line showing the outline of the region-of-interest, an arrow indicating the location of the region-of-interest, or a marker in which only the region-of-interest is replaced with a special-light image may be used.

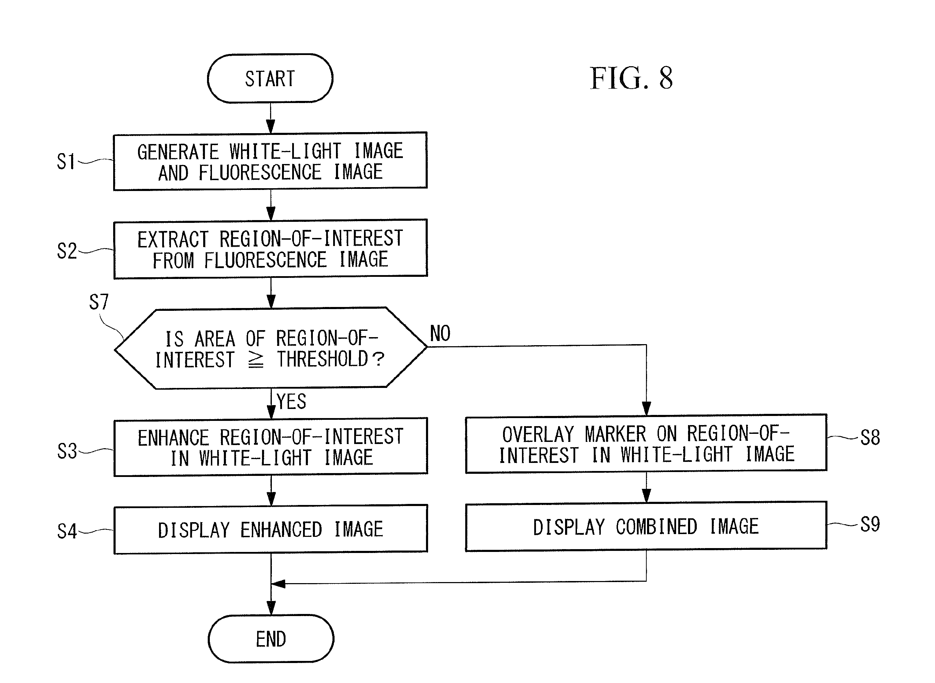

With the thus-configured observation apparatus according to this modification, as shown in Fig. 8, when the region-of-interest is extracted in step S2, the area of the region-of-interest is determined by the determination portion 68. Then, if the area of the region-of-interest is smaller than the prescribed threshold (NO at step S7), the white-light image G1" in which the marker is combined with the region-of-interest (step S8) is displayed on the monitor 7 (step S9). On the other hand, if the area of the region-of-interest is equal to or larger than the prescribed threshold (YES at step S7), the white-light image G1' in which the region-of-interest has been subjected to enhancement processing by the enhancement processing portion 64 is displayed on the monitor 7 (step S4).

In this way, when the region-of-interest is observed from a position that is sufficiently far away, the white-light image G1" in which the region-of-interest is indicated by the marker is displayed on the monitor 7. Accordingly, the user can easily recognize the region-of-interest that exists in the viewing field, no matter how small it is. Then, after the region-of-interest is recognized, the user makes the observation distance sufficiently short by bringing the distal end 2a of the insertion portion 2 close to the region-of-interest, whereupon the white-light image G1" displayed on the monitor 7 is replaced with the white-light image G1'. That is to say, in the white-light image being observed, the region-of-interest is subjected to enhancement processing, whereas the marker disappears. Therefore, the user can perform detailed examination of the region-of-interest. In other words, with this modification, by switching between the images G1' and G1" displayed on the monitor 7 according to the observation distance, an advantage is afforded in that it is possible to show the user an image that is more useful depending on the situation.

In this modification, the determination portion 68 may determine the observation distance by using gradation values of the white-light image G1 instead of the area of the region-of-interest in the fluorescence image G2. The overall brightness of the white-light image G1 increases as the observation distance decreases. Therefore, the determination portion 68 can determine the observation distance by using the gradation values of the white-light image G1, and, similarly to the case where the area of the region-of-interest is used, an image, G1' or G1", that is more useful to the user can be displayed on the monitor 7.

More specifically, the determination portion 68 calculates the mean gradation value of the white-light image G1. Then, when the calculated mean gradation value is larger than a prescribed threshold, the determination portion 68 outputs the white-light image G1 to the enhancement processing portion 64. On the other hand, when the mean gradation value is less than or equal to the prescribed threshold, the determination portion 68 outputs the white-light image G1 to the combining portion 69.

In addition, in this modification, the combining portion 69 may change the display form of the marker depending on the observation distance. For example, the combining portion 69 may increase the transparency of the marker in inverse proportion to the observation distance, that is to say, in proportion to the area of the region-of-interest in the fluorescence image G2 or the mean gradation value of the white-light image G1.

By doing so, as the distal end 2a of the insertion portion 2 is brought closer to the region-of-interest, the marker that is overlaid on the white-light image G1 becomes progressively more transparent and soon disappears. After the marker disappears, the region-of-interest that is subjected to enhancement processing is displayed at that position. Accordingly, an advantage is afforded in that it is possible to switch between the two images G1' and G1" without causing a sense of incongruity in the user who is observing the monitor 7.

### Second Embodiment

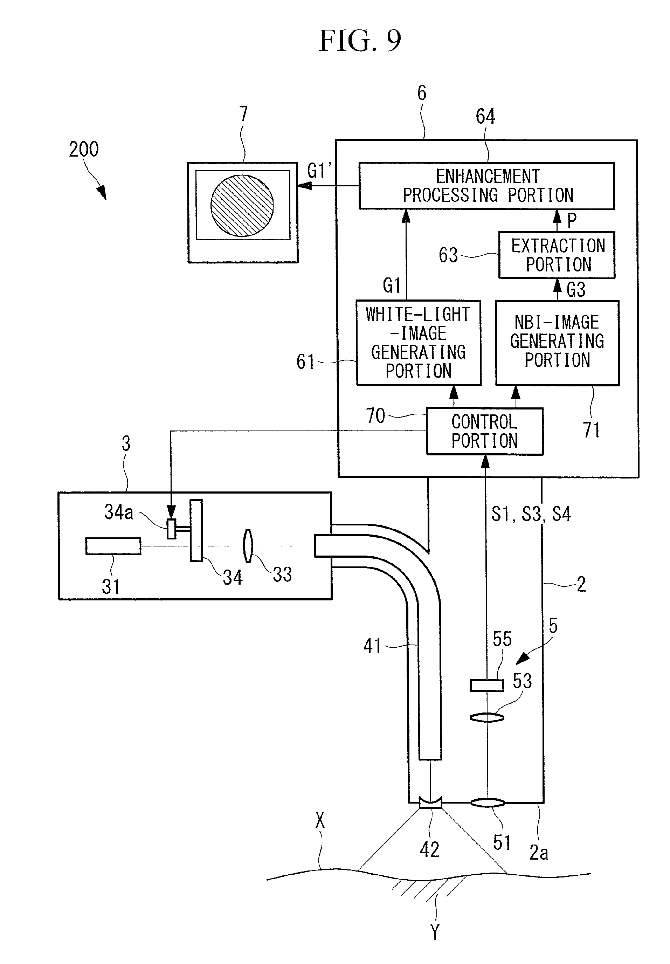

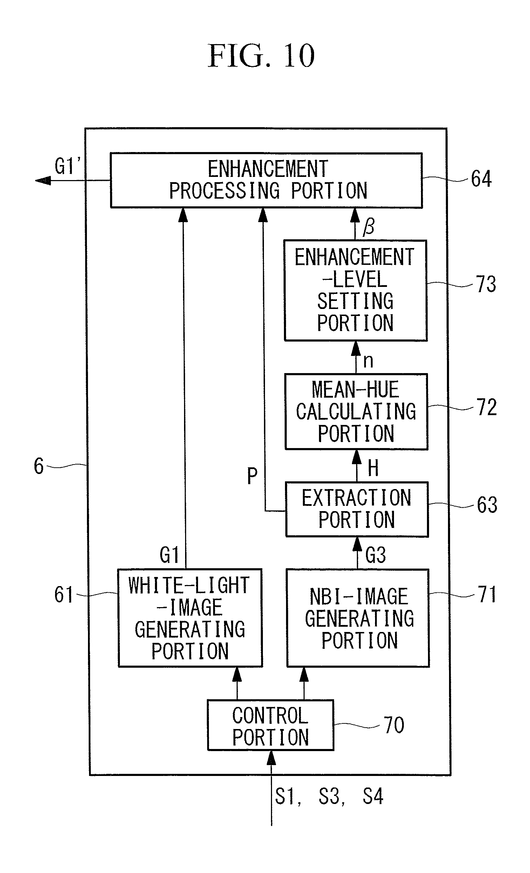

Next, an observation apparatus 200 according to a second embodiment of the present invention will be described with reference to Figs. 9 and 10. In the description of this embodiment, mainly parts that differ from those in the observation apparatus 100 according to the first embodiment described above will be described, and the parts that are common to the observation apparatus 100 will be assigned the same reference signs, and descriptions thereof will be omitted.

The main difference between the observation apparatus 200 according to this embodiment and the observation apparatus 100 according to the first embodiment is that an NBI image G3 is obtained instead of the fluorescence image G2, and a region-of-interest is extracted from the NBI image G3 based on a hue H.

More specifically, as shown in Fig. 9, a light source 3 is provided with a turret 34 having three filters. These three filters pass light in specific wavelength bands from among the light emitted from the xenon lamp 31. Specifically, the three filters selectively transmit white light in a wavelength band of 400 nm to 700 nm, green narrow-band light in a narrow wavelength band having a peak wavelength of 540 nm, and blue narrow-band light having a peak wavelength of 415 nm, respectively. By rotating the turret 34, the white light, the green narrow-band light, and the blue narrow-band light are sequentially input to the illumination unit 4.

The image-acquisition unit 5 includes a single image-acquisition device 55, such as a color CCD, that captures the light collected by the objective lens 51. The image-acquisition device 55 sequentially obtains three types of image information, namely, white-light image information S1, green-light image information S3, and blue-light image information S4, by sequentially irradiating the observation target X with the white light, the green narrow-band light, and the blue narrow-band light from the illumination optical system 42 in the illumination unit 4. Then, the image-acquisition unit 5 outputs the obtained image information S1, S3, and S4 in turn to the image processing unit 6.

The image processing unit 6 includes a control portion 70 that stores the three types of image information S1, S3, and S4 input thereto from the image-acquisition device 55 and an NBI-image generating portion 71 that generates an NBI image G3 from the green-light image information S3 and the blue-light image information S4 stored in the control portion 70.

The control portion 70 controls a motor 34a of the turret 34 so as to assign the white-light image information S1 to the white-light-image generating portion 61 and so as to assign the green-light image information S3 and the blue-light image information S4 to the NBI-image generating portion 71, in synchronization with the switching of the light that is radiated onto the observation target X according to the rotation of the turret 34.

The NBI-image generating portion 71 generates a red-light image from the green-light image information S3, generates a green-light image and a blue-light image from the blue-light image information S4, and generates the NBI image G3 by combining the red-light image, the green-light image, and the blue-light image.

The green narrow-band light and the blue narrow-band light have the property that they are easily absorbed by hemoglobin. In addition, the blue narrow-band light is reflected close to the surface of biological tissue, and the green narrow-band light is reflected at a comparatively deep position in biological tissue. Therefore, in the green-light image and the blue-light image formed by capturing the reflected light (signal light) of the blue narrow-band light from the biological tissue, capillary blood vessels that exist in the outer layer of the biological tissue are clearly captured. On the other hand, in the red-light image formed by capturing the reflected light (signal light) of the green narrow-band light from the biological tissue, thick blood vessels that exist at comparatively deep positions in the biological tissue are clearly captured. In the NBI image G3, in which these two color images are superimposed, a lesion Y such as a squamous cell carcinoma takes on a dark brown color.

The extraction portion 63 extracts a region-of-interest based on the hue H of the NBI image G3. Here, the hue H is one of the properties of a color (hue, saturation, lightness) and is an aspect of color (for example, red, blue, yellow) represented by a numerical value in the range 0 to 360 using the so-called Munsell color wheel. More specifically, the extraction portion 63 calculates the hue H of each pixel in the NBI image G3 and extracts pixels having a dark-brown color (for example, a hue H of 5 to 35) as the region-of-interest.

Next, the operation of the thus-configured observation apparatus 200 will be described.

To observe biological tissue inside a body, that is, the observation target X, using the observation apparatus 200 according to this embodiment, as in the first embodiment, the insertion portion 2 is inserted inside the body, and the light source 3 is operated. The white light, the green narrow-band light, and the blue narrow-band light from the light source 3 are sequentially radiated onto the observation target X via the coupling lens 33, the light guide fiber 41, and the illumination optical system 42.

In the observation target X, the white light, the green narrow-band light, an the blue narrow-band light are sequentially reflected and are collected by the objective lens 51. The white light, the green narrow-band light, and the blue narrow-band light collected by the objective lens 51 are obtained in the form of the white-light image information S1, the green-light image information S3, and the blue-light image information S4, respectively. The image information S1, S3, and S4 obtained by the image-acquisition device 55 are then sent to the image processing unit 6.

In the image processing unit 6, the image information S1, S3, and S4 are stored in the control portion 70. Next, the white-light image information S1 is input to the white-light-image generating portion 61, where the white-light image G1 is generated. Also, the green-light image information S3 and the blue-light image information S4 are input to the NBI-image generating portion 71, where the NBI image G3 is generated. The generated NBI image G3 is sent to the extraction portion 63, where a region-of-interest having a dark-brown color is extracted. Subsequently, a white-light image G1' in which the region-of-interest is subjected to enhancement processing is displayed on the monitor 7, as in steps S3 and S4 in the first embodiment.

Thus, with the observation apparatus 200 according to this embodiment, by using the NBI image G3 as the special-light image, the region-of-interest is extracted based on the hue H. By doing so, as in the first embodiment, an advantage is afforded in that it is possible to show the user the white-light image G1' in which the region-of-interest can be easily distinguished and the morphology of the region-of-interest can be confirmed in detail.

The individual modifications described in the first embodiment can also be suitably employed in this embodiment.

### Modification

Next, a modification of the observation apparatus 200 according to the second embodiment will be described.

The observation apparatus according to this modification is one in which the image processing unit 6 in the observation apparatus 200 is modified; as shown in Fig. 10, a mean-hue calculating portion 72 and an enhancement-level setting portion 73 are further provided in the image processing unit 6.

In this modification, the extraction portion 63 outputs the positions P of pixels constituting the region-of-interest to the enhancement processing portion 64, and outputs the hues H of those pixels to the mean-hue calculating portion 72.

The mean-hue calculating portion 72 calculates the mean n of the hues H of the pixels constituting the region-of-interest, which were extracted by the extraction portion 63. Then, the mean-hue calculating portion 72 outputs the calculated mean n of the hues H to the enhancement-level setting portion 73.

The enhancement-level setting portion 73 sets a degree of enhancement processing, β, in the enhancement processing portion 64 on the basis of the mean n of the hues H input thereto from the mean-hue calculating portion 72. More specifically, the enhancement-level setting portion 73 holds a table in which the mean n of the hues H and the degree of enhancement processing, β, are associated with each other. This table is set so that, for example, the degree of enhancement processing, β, increases as the mean n of the hues H approaches red or yellow, which are located on either side of dark-brown in the color wheel. The enhancement-level setting portion 73 derives the degree of enhancement processing, β, corresponding to the mean n of the hues H from the table and outputs the derived degree β to the enhancement processing portion 64.

The enhancement processing portion 64 executes enhancement processing on a region in the white-light image G1 corresponding to the position P of the region-of-interest input thereto from the extraction portion 63, using the degree β input thereto from the enhancement-level setting portion 73.

With the thus-configured observation apparatus according to this modification, once the region-of-interest is extracted in the extraction portion 63, the mean n of the hues H of that region-of-interest is calculated in the mean-hue calculating portion 72. Then, the degree of enhancement processing, β, is determined in the enhancement-level setting portion 73 on the basis of the calculated mean n of the hues H, and the region-of-interest in the white-light image G1 is subjected to enhancement processing in the enhancement processing portion 64 with the determined degree β.

In this way, by determining the degree of enhancement processing, β, by considering the hues H of the entire region-of-interest, when the hues H of the region-of-interest approach red or yellow, the region-of-interest is more strongly enhanced. Accordingly, even for a region-of-interest in which the difference in morphology of the tissue is small relative to the surrounding region, as in an early-stage lesion Y, for example, it can be displayed in a manner sufficiently enhanced with respect to the surrounding region, which affords the advantage that it can be reliably recognized by the user.

The table that associates the mean n of the hues H and the level of enhancement processing is set so that the degree of enhancement processing, β, increases as the mean n of the hues H approaches dark-brown in the color wheel. With this function, when the hue H of the region-of-interest is close to dark-brown, the region-of-interest is more strongly enhanced. Accordingly, an advantage is afforded in that a region-of-interest with a high concentration of blood vessels can be reliably recognized by the user.

### Third Embodiment

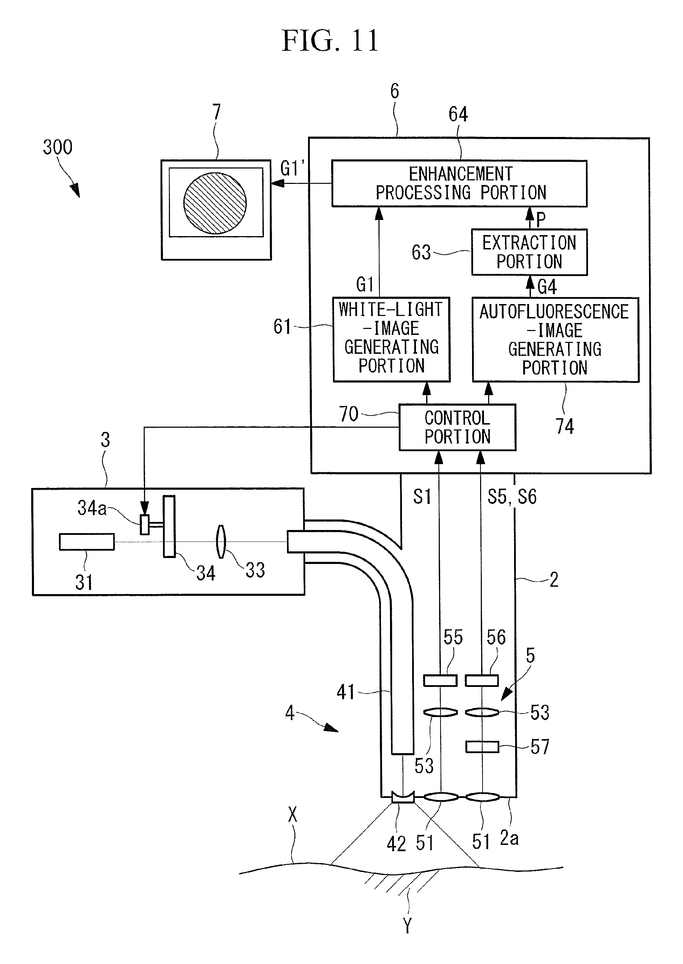

Next, an observation apparatus 300 according to a third embodiment of the present invention will be described with reference to Fig. 11.

In the description of this embodiment, mainly parts that differ from those in the observation apparatus 100 according to the first embodiment described above will be described, and parts that are common to the observation apparatus 100 will be assigned the same reference signs, and descriptions thereof will be omitted.

The main difference between the observation apparatus 300 according to this embodiment and the observation apparatus 100 is that it obtains an autofluorescence image G4 instead of the fluorescence image G2, and extracts a region-of-interest from the autofluorescence image G4 on the basis of the hue H.

More specifically, as shown in Fig. 11, the light source 3 is provided with a turret 34 having three filters. These three filters pass light in specific wavelength bands from among the light emitted from the xenon lamp 31. Specifically, the three filters selectively transmit white light in a wavelength band of 400 nm to 700 nm, green reference light having a peak wavelength of 550 nm, and blue excitation light having a peak wavelength of 400 nm, respectively. By rotating the turret 34, the white light, the reference light, and the excitation light are sequentially input to the illumination unit 4.

The image acquisition unit 5 is a binocular system that obtains white-light image information S1 and autofluorescence image information S5 and S6 with separate optical systems. In other words, the image-acquisition unit 5 includes two optical systems each having an objective lens 51 that collects light coming from the observation target X, a focusing lens 53 that focuses the light emerging from the objective lens 51, and an image-acquisition device 55 or 56 that captures the light focused by the focusing lens 53. These two optical systems are provided side-by-side at the distal end of the insertion portion 2.

The first optical system obtains the white-light image information S1 with the image-acquisition device 55, such as a color CCD.

The second optical system further includes an excitation-light cutting filter 57 between the objective lens 51 and the focusing lens 53 and obtains the autofluorescence image information S5 and S6 by capturing autofluorescence emitted from the observation target X and green return light with the image-acquisition device 56, such as a high-sensitivity monochrome CCD. In this embodiment, the excitation-light cutting filter 57 selectively transmits light in a wavelength band of 500 to 630 nm, corresponding to the autofluorescence of the observation target X and the green return light, and blocks the excitation light.

By sequentially irradiating the observation target X with the white light, the reference light, and the excitation light from the illumination optical system 42 in the illumination unit 4, the image-acquisition devices 55 and 56 sequentially obtain three types of image information, namely, the white-light image information S1, first autofluorescence image information S5, and second autofluorescence image information S6. Then, each of the image-acquisition devices 55 and 56 outputs the obtained image information S1, S5, and S6 in turn to the image processing unit 6.

The image processing unit 6 includes a control portion 70 that stores the three types of image information S1, S5, and S6 obtained by the image-acquisition devices 55 and 56 and an autofluorescence-image generating portion 74 that generates an autofluorescence image G4 from the first autofluorescence image information S5 and the second autofluorescence image information S6 stored in the control portion 70.

The control portion 70 controls the motor 34a of the turret 34 so as to assign the white-light image information S1 to the white-light-image generating portion 61 and so as to assign the first autofluorescence image information S5 and the second autofluorescence image information S6 to the autofluorescence-image generating portion 74, in synchronization with the switching of the light that is radiated onto the observation target X according to the rotation of the turret 34.

The autofluorescence-image generating portion 74 generates the first autofluorescence image from the first autofluorescence image information S5 and generates the second autofluorescence image from the second autofluorescence image information S6. At this time, the autofluorescence-image generating portion 74 pseudo-colors the first autofluoresence image with red and blue and pseudo-colors the second autofluorescence image with green. Then, the autofluorescence-image generating portion 74 generates a color autofluorescence image G4 by combining the pseudo-colored first autofluorescence image and second autofluorescence image. In the autofluorescence image G4, the lesion Y is displayed as a red-violet (for example, a hue H from 300 to 350) region.

The extraction portion 63 extracts the region-of-interest based on the hues H of the autofluorescence image G4. More specifically, the extraction portion 63 calculates the hue H of each pixel of the autofluorescence image G4 and extracts pixels having a red-violet color (for example, a hue H of 300 to 350) as a region-of-interest.

Next, the operation of the thus-configured observation apparatus 300 will be described.

To observe biological tissue inside a body, that is, the observation target X, using the observation apparatus 300 according to this embodiment, the observation target is sequentially irradiated with the white light, the reference light, and the excitation light, similarly to the second embodiment.

The white light is reflected at the surface of the observation target X. On the other hand, the excitation light excites a substance contained in the observation target X, thereby emitting autofluorescence from the observation target X. The white light collected by the first objective lens 51 is obtained in the form of the white-light image information S1 by the image-acquisition device 55. The reference light and the autofluorescence collected by the second objective lens 51 are respectively obtained in the form of the first autofluorescence image information S5 and the second autofluorescence image information S6 by the image-acquisition device 56. The image information S1, S5, and S6 obtained by the image-acquisition devices 55 and 56 are sent to the image processing unit 6.

In the image processing unit 6, the image information S1, S5, and S6 are stored in the control portion 70. Then, the white-light image information S1 is input to the white-light-image generating portion 61, where the white-light image G1 is generated. On the other hand, the first autofluorescence image information S5 and the second autofluorescence image information S6 are input to the autofluorescence-image generating portion 74, where the autofluorescence image G4 is generated. The generated autofluorescence image G4 is sent to the extraction portion 63, where a region-of-interest having a red-violet color is extracted. Subsequently, the white-light image G1' in which the region-of-interest is subjected to enhancement processing is displayed on the monitor 7, similarly to steps S3 and S4 in the first embodiment.

In this way, with the observation apparatus 300 according to this embodiment, the region-of-interest is extracted on the basis of the hue H, using the autofluorescence image G4 as a special-light image. In this way, too, as with the first embodiment, an advantage is afforded in that it is possible to show the user the white-light image G1' in which the region-of-interest can be easily distinguished and the morphology of the region-of-interest can be confirmed in detail.

The individual modifications described in the first embodiment and the second embodiment can also be suitably employed in this embodiment.

### Fourth Embodiment

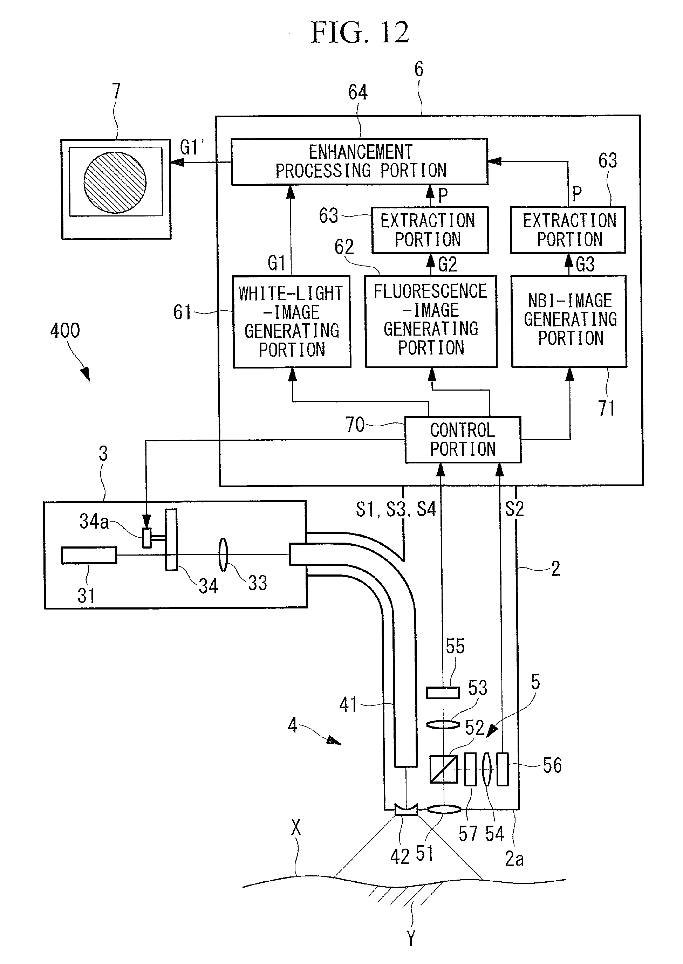

Next, an observation apparatus 400 according to a fourth embodiment of the present invention will be described with reference to Fig. 12.

The observation apparatus 400 according to this embodiment is a combination of the first embodiment and the second embodiment. Therefore, in the description of this embodiment, parts that are common to the first embodiment and the second embodiment are assigned the same reference signs, and descriptions thereof will be omitted.

As shown in Fig. 12, the light source 3 includes a turret 34 having three filters. These three filters pass light in specific wavelength bands from among the light emitted from the xenon lamp 31. In this embodiment, one of the three filters is the same as the filter 32 in the first embodiment and selectively transmits the excitation light and the white light. The other two filters are the same as two of the filters in the second embodiment and selectively transmit green narrow-band light and blue narrow-band light, respectively. By rotating the turret 34, the excitation light and white light, the green narrow-band light, and the blue narrow-band light are sequentially input in a time-division manner to the illumination unit 4.

The image processing unit 6 includes both the fluorescence-image generating portion 62 and the NBI-image generating portion 71. Furthermore, the image processing unit 6 includes two extraction portions 63 that extract regions-of-interest from the fluorescence image G2 and the NBI image G3, respectively. The first extraction portion 63 extracts the region-of-interest on the basis of gradation values from the fluorescence image G2 input thereto from the fluorescence-image generating portion 62, similarly to the extraction portion 63 in the first embodiment. The second extraction portion 63 extracts the region-of-interest on the basis of hues H from the NBI image G3 input thereto from the NBI-image generating portion 71, similarly to the extraction portion 63 in the second embodiment.

The enhancement processing portion 64 compares the positions P in the two regions-of-interest received from each of the extraction portions 63 and executes enhancement processing on the region that is common to these two regions-of-interest.

With the thus-configured observation apparatus 400 according to this embodiment, by using the fluorescence image G2 and the NBI image G3 as special-light images, a region that is common to the two regions-of-interest extracted from these two special-light images serves as the final region-of-interest. Accordingly, since the region-of-interest, such as a lesion Y in the observation target X, is more accurately extracted, the user can more-accurately recognize the position of the region-of-interest.

The individual modifications described in the first embodiment and the second embodiment can also be suitably employed in this embodiment.

### {Reference Signs List}

- 100, 200, 300, 400: Observation apparatus
- 2: Insertion portion
- 2a: Distal end
- 3: Light source
- 31: Xenon lamp
- 32: Filter
- 33: Coupling lens
- 34: Turret
- 4: Illumination unit
- 41: Light guide fiber
- 42: Illumination optical system
- 5: Image-acquisition unit
- 51: Objective lens
- 52: Dichroic mirror
- 53, 54: Focusing lens
- 55, 56: Image-acquisition device
- 57: Excitation-light cutting filter
- 6: Image processing unit
- 61: White-light-image generating portion (return-light-image generating portion)
- 62: Fluorescence-image generating portion (special-light-image generating portion)
- 63: Extraction portion
- 64: Enhancement processing portion
- 65: Division portion
- 66: Mean-gradation-value calculating portion
- 67: Enhancement-level setting portion
- 68: Determination portion (display switching portion)
- 69: Combining portion
- 70: Control portion
- 71: NBI-image generating portion (special-light-image generating portion)
- 72: Mean-hue calculating portion
- 73: Enhancement-level setting portion
- 74: Autofluorescence-image generating portion (special-light-image generating portion)
- G1: White-light image (return-light image)
- G2: Fluorescence image (special-light image)
- G2': Division image
- G3: NBI image (special-light image)
- G4: Autofluorescence image (special-light image)
- X: Observation target
- Y: Lesion

## Claims

1. An observation apparatus comprising:
a light source that irradiates a subject with illumination light and special light in a wavelength band different from that of the illumination light, which acts on a specific region of the subject;
a return-light-image generating portion that generates a return-light image by capturing return light emitted from the subject due to irradiation with the illumination light from the light source;
a special-light-image generating portion that generates a special-light image by capturing signal light emitted from the subject due to irradiation with the special light from the light source;
an extraction portion that extracts the specific region from the special-light image generated by the special-light-image generating portion; and
an enhancement processing portion that performs enhancement processing, which is based on return-light image information, on the return-light image generated by the return-light-image generating portion, in a region corresponding to the specific region extracted by the extraction portion.

2. The observation apparatus according to claim 1, wherein the enhancement processing portion enhances the contrast of at least one of structure and color.

3. The observation apparatus according to claim 1 or claim 2, wherein, as the special light, the light source radiates excitation light that excites a fluorescent substance contained in the specific region, and
the special-light-image generating portion generates, as the special-light image, a fluorescence image by capturing fluorescence from the fluorescent substance.

4. The observation apparatus according to one of claims 1 to 3, wherein the extraction portion extracts, as the specific region, a region having a gradation value equal to or higher than a prescribed threshold.

5. The observation apparatus according to claim 4, further comprising an enhancement-level setting portion that sets, for the specific region, a degree of enhancement to be performed by the enhancement processing portion, on the basis of the gradation value of the specific region extracted by the extraction portion.

6. The observation apparatus according to claim 1 or claim 2, wherein the light source radiates narrow-band light as the special light, and
the special-light-image generating portion generates, as the special-light image, a narrow-band-light image by capturing return light from the subject by irradiation with the narrow-band light.

7. The observation apparatus according to claim 1 or claim 2, wherein the light source radiates, as the special light, excitation light that excites autofluorescence in a substance contained in the subject, and
the special-light-image generating portion generates, as the special-light image, an autofluorescence image by capturing autofluorescence from the substance.

8. The observation apparatus according to one of claims 1, 2, 6, and 7, wherein the extraction portion extracts, as the specific region, a region having a prescribed hue.

9. The observation apparatus according to claim 8, further comprising an enhancement-level setting portion that sets, for the region, a degree of enhancement to be performed by the enhancement processing portion on the basis of a hue of the specific region extracted by the extraction portion.

10. The observation apparatus according to one of claims 1 to 9, further comprising:
a combining portion that combines a marker showing the specific region extracted by the extraction portion with the return-light image generated by the return-light-image generating portion;
a display portion that displays an image;
a determination portion that determines an observation distance to the subject; and
a display switching portion that selectively displays, on the display portion, a return-light image in which the specific region is enhanced by the enhancement processing portion and a return-light image with which the marker is combined by the combining portion, on the basis of the observation distance determined by the determination portion.

11. The observation apparatus according to claim 10, wherein the determination portion determines the observation distance by using a gradation value of the return-light image generated by the return-light-image generating portion.

12. The observation apparatus according to claim 10, wherein the determination portion determines the observation distance by using an area, in the special-light image, of the specific region extracted by the extraction portion.
